# EUROPEAN PATENT SPECIFICATION

(11) **EP 2 953 586 B1**
(45) Date of publication and mention of the grant of the patent: **08.04.2020**
(21) Application number: 14707021.3
(22) Date of filing: 06.02.2014
(51) Int. Cl.: A61F 2/46, A61F 2/30

(54) **APPARATUS FOR POSITIONING A PROSTHESIS**
VORRICHTUNG ZUR POSITIONIERUNG EINER PROTHESE
APPAREIL POUR POSITIONNER UNE PROTHÈSE

(30) Priority: 08.02.2013 US 201313762646
(43) Date of publication of application: 16.12.2015
(73) Proprietor: Biomet Manufacturing, LLC, Warsaw, IN 46582 (US)
(72) Inventor: WITT, Tyler D., Warsaw, Indiana 46582 (US); DAVENPORT, Austen, Columbia City, Indiana 46725 (US)
(74) Representative: Mays, Julie
(86) International application number: PCT/US2014/015064
(87) International publication number: WO 2014/124115

(56) References cited:
- EP-A1- 2 572 679
- FR-A1- 2 994 645
- US-A- 5 904 688
- US-A1- 2003 187 512
- US-A1- 2005 137 603
- US-A1- 2007 005 144

## Description

### FIELD

The subject disclosure relates to an instrument and method for using an instrument to position a prosthesis, and particularly to an instrument operable in a plurality of configurations and/or orientations to position and remove portions of the prosthesis system.

### BACKGROUND

This section provides background information related to the present disclosure which is not necessarily prior art.

A member can be positioned in a subject for performing or recreating an interaction of various portions of a subject. For example, a prosthesis can be positioned in a subject anatomy, such as a human anatomy, to replace or repair damaged portions of the subject anatomy. For example, an acetabular prosthesis can be positioned in an acetabulum of a subject to replace a natural acetabulum. The prosthetic acetabulum can include various portions that replace and mimic the natural anatomy to allow for articulation with a femur. The femur can be the natural femur or a prosthetic proximal femoral member. An acetabular prosthesis can be positioned within a subject using an assembly or insertion tool such as that disclosed in US 2005/137603.

### SUMMARY

This section provides a general summary of the disclosure, and is not a comprehensive disclosure of its full scope or all of its features.

An acetabular prosthesis can be positioned within a subject using an assembly or insertion tool. The insertion tool can include a multi-use tool that can be used to engage and position an acetabular shell, a bearing liner (also referred to alone as a liner herein) for insertion within the acetabular shell, and a removal of a liner positioned within the acetabular shell. It is understood that the multi-use tool can include features to engage the shell and/or liner to perform any function including one or more functions by orienting the multi-use tool relative to the shell and/or the liner.

The invention is defined according to claim 1. A multi-use tool can include a member having a first surface and a second surface. The first surface can engage a first portion of the shell and/or liner. The second surface can engage a second portion of a shell and/or liner for insertion and/or removal of the liner or the shell.

According to various embodiments, the multi-use tool can include a handle engagement portion near a center and a shell engaging portion near an outer edge of an annular member. The annular member can be substantially solid or formed as a ring with a central hub and spoke extending from the central hub to the ring. The outer ring can include engaging tabs or projections to engage the shell for positioning the shell and/or engaging the shell to release a liner. Additionally, the multi-use tool can include a surface, such as on the spokes projecting from the hub, to contact a liner for inserting the liner into the shell. Additionally, the multi-use tool can include a second shell engaging region to align the multi-use tool relative to the shell to assist in aligning the liner relative to the shell.

In addition to the multi-use tool, various handles and additional instruments can be provided to cooperate with the multi-use tool. The additional portions can assist in positioning the shell relative to a subject, a liner relative to the shell, and remove a liner from the shell after being positioned in the shell. For example, a revision procedure may require removal of a liner from a shell while maintaining the shell in the subject. Revision procedures can include instances where the liner is damaged. Revision can also include replacement of the proximal femoral component requiring a different size or configuration of the liner.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
Fig. 1A is a perspective view of a first side of a multi-use instrument;
Fig. 1B is a perspective view of a second side of the multi-use instrument;
Fig. 2 is an exploded view of an assembly for insertion of an acetabular shell with the multi-use tool;
Fig. 3 is a partial cross-section view of an assembly of the multi-use tool onto an acetabular shell, according to various embodiments;
Fig. 4 is an exploded view of the multi-use tool and an insertion handle for positioning a liner within a shell;
Fig. 5 is a cross-section view of a liner insertion handle and a liner being positioned on a shell;
Fig. 6 is a cross-section view of the insertion handle with an engagement member disengaging a liner;
Fig. 7 is an exploded view of the multi-use tool in an assembly for forming a pilot hole in a liner;
Fig. 8 is a detailed cross-section view of a multi-use tool guiding a drill bit into a liner;
Fig. 9 is a detailed cross-section view of a multi-use tool after the pilot hole has been formed;
Fig. 10 is a perspective view of a liner removal tool; and
Fig. 11 is a view of the liner removal tool engaging and removing a liner from a shell.

Corresponding reference numerals indicate corresponding parts throughout the several views of the drawings.

### DETAILED DESCRIPTION

Example embodiments will now be described more fully with reference to the accompanying drawings.

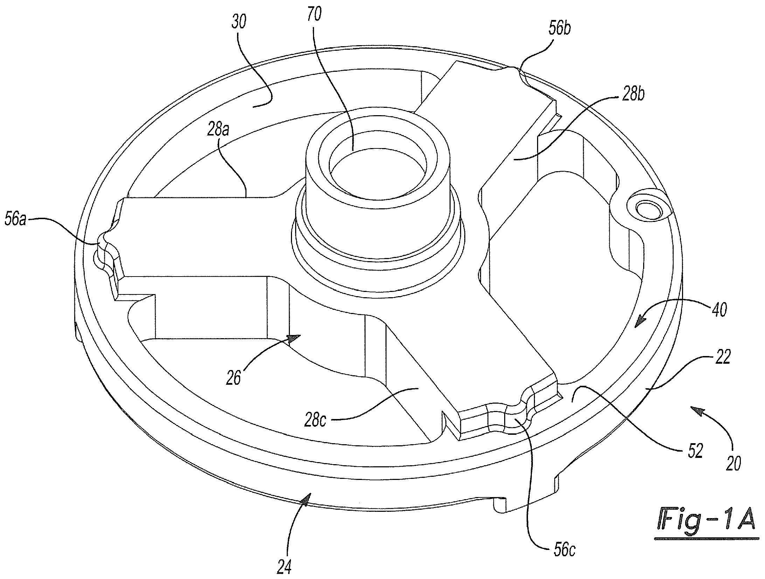

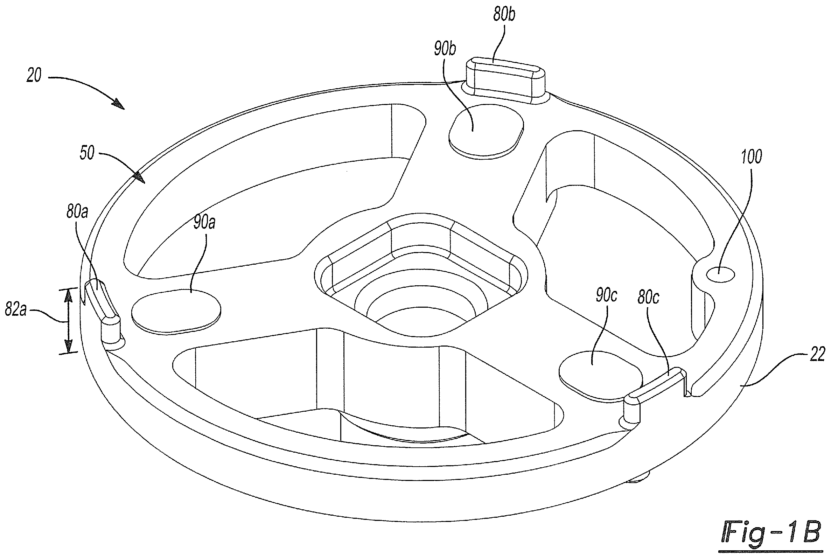

According to various embodiments, a multi-use tool 20, as illustrated in Figs. 1A and 1B may be used and provided for performing various interactions with an acetabular shell (as discussed herein) and a liner to be positioned within the acetabular shell (as discussed herein). The multi-use tool 20 may generally be provided to have an exterior geometry or a selected geometry that substantially matches at least one of an acetabular shell or an acetabular liner. As exemplarily illustrated, the multi-use tool 20 has an external wall 22 that is substantially annular. The external wall 22 is an exterior wall surface of an annular ring 24 that extends around the multi-use tool 20. Positioned internal to the ring 24 is a central hub 26 and one or more spokes or arms 28a, 28b, and 28c that extend from the central hub 26 to an internal wall surface 30 of the ring 24. The multi-use tool 20, including the ring 24, the central hub 26, and the arms 28a-28c, may define a first side, as illustrated in Fig. 1A, which can be an acetabular cup engaging side 40. A second side 50 of the multi-use tool 20, as illustrated in Fig. 1B, can be a liner engaging or positioning side 50. As discussed herein, the multi-use tool 20 can engage various portions of an acetabular shell, a liner, and various handles or insertion handles, for positioning and/or removing at least one of the acetabular shell or the acetabular liner. The multi-use tool 20 can, therefore, be used for a plurality of purposes during a procedure to either place an acetabular shell and/or liner or remove at least one of the acetabular shell and/or liner.

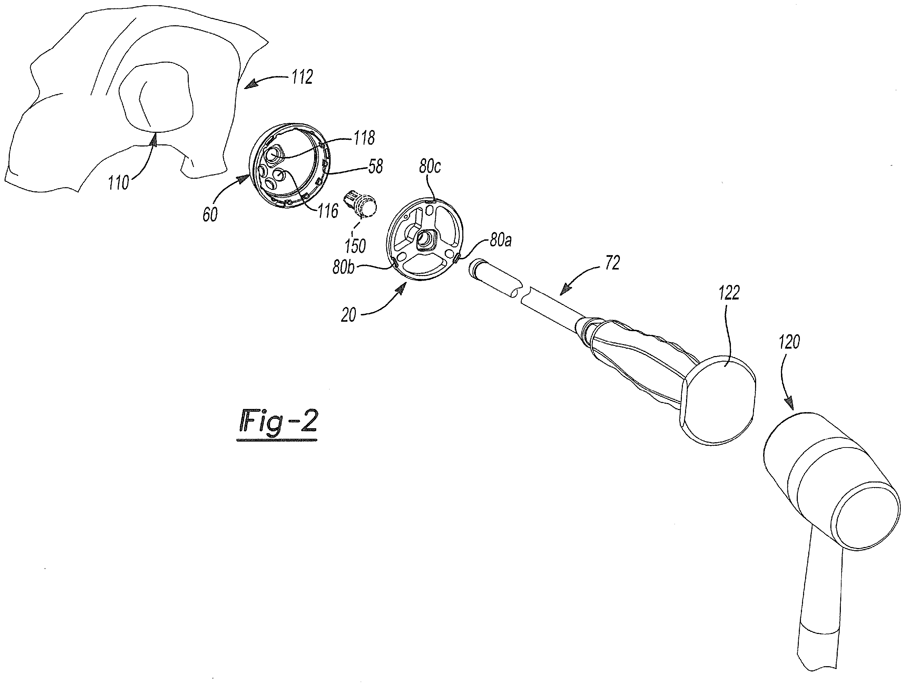

The shell engaging side 40 can include a shell facing surface 52 which may or may not contact an acetabular shell, as discussed herein. An anti-rotation tab 56a, 56b, and 56c can be formed to extend from a raised portion of each of the respective arms 28a-28c. Each of the anti-rotation tabs 56a-56c can engage at least one anti-rotation scallop or recess 58 of a acetabular shell 60, as illustrated in Fig. 2. As discussed further herein, the anti-rotation tab 56a-56c can engage respective recesses 58 of the acetabular shell 60 to rotationally fix the shell 60 relative to the multi-use tool 20. The anti-rotation tabs 56a-56c can be positioned within the respective recesses 58 at any selected orientation. That is, the multi-use tool 20 can be rotated relative to the shell 60 and the anti-rotation tabs 56a-56c can register in any selected three scallop recesses 58 of the shell 60. Therefore, the multi-use tool 20 can be efficiently and quickly positioned relative to the shell 60 in any selected rotational position. Once the shell 60 is positioned relative to the multi-use tool such that the anti-rotation tabs 56a-56c are engaged or received within the scallop recesses 58 the shell 60 is unable to rotate relative to the multi-use tool 20. It is understood, however, that the shell 60 and the multi-use tool 20 can include a keyed portion such as the multi-use tool 20 will engage the shell 60 in only a single orientation.

After positioning the multi-use tool 20 relative to the shell 60, the anti-rotation tabs 56a-56c contact an internal surface of the scallop recess 58. The scallop recesses can include those such as in the acetabular shell used with the RINGLOC® orthopedic prosthesis system sold by Biomet, Inc. having a place in Warsaw, Indiana. Additionally, a surface of the arm facing the cup 60 can engage near the scallop recesses 58 to provide a surface area for impacting the shell 60 into a patient. According to various embodiments, the surface 52 of the ring 24 need not directly contact the shell 60. It is understood, however, that the surface 52 of the ring 24 can engage the shell 60 if selected.

The central hub 26 can also include a threaded boss 70 that can have internal and/or external threads to engage a handle 72. The handle 72 can have internal or external threads to engage the threaded boss 70. Accordingly, the multi-use tool 20 can be axially fixed relative to the handle 72 for use of the multi-use tool relative to the shell 60. Additionally, the handle 72 can be rotationally fixed relative to the multi-use tool 20 by a locking feature or locking thread, such as a counter-threaded nut to fix the handle 72 relative to the multi-use tool 20.

The multi-use tool 20, as illustrated in Fig. 1B, can also include a bearing facing an engaging side. The bearing engaging side 50 can include one or more shell interface tabs 80a, 80b, and 80c. The tabs 80a-80c can engage recesses in the shell 60, as discussed further herein. The tabs 80a-80c are illustrated as being substantially aligned with the arms 28a-28c, but it is understood that this is not required.

The tabs 80a-80c can include dimensions that are appropriate for engaging recesses in the shell 60. For example, a length and width of the tabs 80a-80c can be provided to engage the shell 60 in a manner to allow for a vibrational force to be transmitted to the shell, as discussed further herein. Moreover, the tabs 80a-80c can include a height to allow the tabs 80a-80c to bottom out within the respective recesses 310 as discussed further herein.

Formed on the liner facing side 50 can be one or more non-metallic buffer members 90a, 90b, and 90c. As discussed herein, a liner can be positioned against the buffers 90a-90c to limit or eliminate contact with other portions of the multi-use tool 20 and, particularly, the material that forms the remainder of the multi-use tool 20. The multi-use tool 20 can be formed of a material, such as a metal or metal alloy, and the buffers 90a-90c can be formed of a non-metallic material, such as a selected polymer, carbon material, fabric, etc. The non-metallic buffers 90a-90c can substantially eliminate scratching or transfer of metal particles between the liner and the multi-use tool 20. Therefore, the buffers 90a-90c can eliminate or substantially minimize scratching of the liner by the multi-use tool 20. This can also eliminate or substantially reduce wear debris that may be created due to contact between the multi-use tool 20 and a respective liner.

The multi-use tool 20 can further include a guide hole or guide-bore 100. The guide-bore 100 is formed through the ring 24 of the multi-use tool 20. As discussed further herein, the guide-bore 100 can be used to form a pilot hole or guide hole in a liner to assist in removal of the liner from the acetabular shell 60. According to various embodiments, the guide-bore 100 can be formed at an angle, as discussed and illustrated further herein, relative to a central axis of the multi-use tool 20.

With continuing reference to Figs. 1A and 1B, and additional reference to Fig. 2, the multi-use tool can be used in one or more manners to insert the shell 60. The shell 60 can be positioned within a pelvis 112 of a patient after selected preparation of an acetabulum 110 of the patient. As is generally understood in the art, the acetabulum 110 of the pelvis 112 can be prepared such as by reaming, resection, and the like. After appropriate preparation of the acetabulum 110, the shell 60 can be positioned within the acetabulum 110 in a selected manner. As is generally known in the art, fixation of the acetabular shell 60 can be made with the acetabulum 110. For example, the acetabular shell 60 can include at least one throughbore or passage hole 116 for allowing a screw to pass through the shell 60 to engage the acetabulum 110. Additionally, the shell 60 can include an apical depression or throughbore 118 to assist in engaging an insertion tool.

According to various embodiments, the multi-use tool 20 may engage the anti-rotation scallops 58 of the shell 60 with the anti-rotation tabs 56a-56c. The anti-rotation tabs 56a-56c fix the shell 60 rotationally relative to the multi-use tool 20. The handle or insertion rod 72 can threadably engage the multi-use tool 20 to axially and rotationally fix the multi-use tool 20 relative to the insertion rod 72. The interconnection of the insertion rod 72 with the multi-use tool 20 allows for the multi-use tool 20 to hold the shell 60 in a selected orientation relative to the acetabulum 110 during an insertion. During an insertion or implantation, the insertion rod 72 can be impacted with an impaction hammer 120 on an impaction end 122, as is generally known in the art. The multi-use tool 20, however, can assist in holding the shell 60 in a selected orientation and/or location during of the tool 20 impaction with the impaction tool 120.

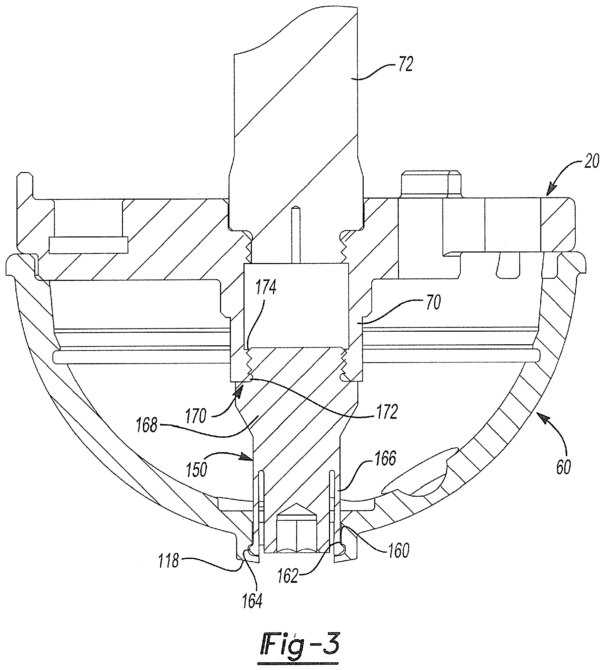

With continuing reference to Figs. 1A-2, and additional reference to Fig. 3, a quick connect member 150 can also be provided to engage the shell 60. The engagement of the quick connect member 150 with the shell 60 can be at the apical hole 118. The apical hole may include a ridge or groove 160 in a side wall 162 to receive projections or fingers 164 that extend from deflectable legs 166. In one example, the ridge or groove can include an internal thread in the throughbore 118. An appropriate number of the deflectable legs 166 can be formed to deflect relative to a body 168 during positioning of the quick insertion member 150 into the apical hole to engage the groove 160. Spaced away from the deflectable legs 160 can be a multi-use tool connection section 170. The multi-use tool connection section 170 that can include a groove 172 formed in a wall 174 of the quick connect member 150. A deflectable or connection portion with the multi-use tool 20 at the hub 70 can engage the quick connect member 150. Alternatively, or in addition to, the quick connect region of the multi-use tool hub 70 can be a threaded interconnection between the quick connect member 150 and the hub 70.

Accordingly, via the quick connect member 150, the multi-use tool 20 may engage the shell 60. When the quick connect member 150 has engaged both the multi-use tool 20 and the shell 60, the shell 60 is also axially fixed relative to the multi-use tool 20. Therefore, with the quick connect member 150 in place, when the insertion rod 72 is fixed to the multi-use tool 20, the insertion rod 72 is also axially fixed relative to the shell 60. With the quick connect member 150, the rod 72 can be both axially and rotationally fixed to the shell 60 by interconnection with the multi-use tool 20. As discussed above, the multi-use tool 20 includes the anti-rotation tabs 56a-56c to rotationally fix the shell relative to the multi-use tool 20 and to the rod 72. The quick connect member 150 may then axially fix the shell 60 relative to the multi-use tool 20, and in turn to the insertion rod 72, when the quick connect member 150 is used. It is understood, however, that the quick connect member 150 is not required to engage the shell 60 for insertion of the shell 60. The multi-use tool 20 alone can then directly engage the shell 60 to rotationally fix the shell 60 relative to the multi-use tool 20, and then, in turn, to the rod 72. The user can determine the amount of fixation and limitation of motion between the shell 60 and the rod 72 by selection of use of the multi-use tool 20 alone, or the multi-use tool 20 used with the quick connect member 150.

The shell 60 can have inserted therein a liner that is configured to articulate with a portion of a proximal femur, such as a natural proximal femur or a prosthetic proximal femur. The liner can be formed of a substantially hard and rigid material, including a ceramic material and/or a metal material. For example, a liner formed of ceramic in the C2a-Taper™ prosthesis system sold by Biomet, Inc. can be inserted into the shell 60. A metal liner can include the cobalt-chromium alloy liner, such as that in the M2A™- metal on metal articulation system, sold by Biomet, Inc., can also be positioned within the shell 60 to articulate with a selected proximal femur portion.

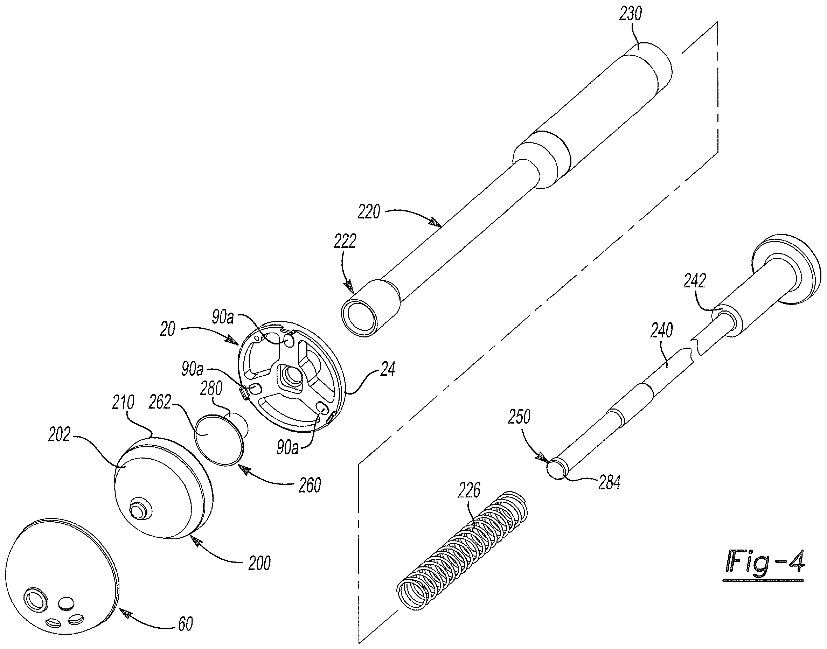

With continuing reference to Figs. 1 and 3, and additional reference to Fig. 4, a liner formed of a hard material, referenced herein as a hard-liner 200 is illustrated. The hard-liner 200 can include a male taper 202 to engage a female taper (not specifically illustrated here) in the shell 60 to fix or engage the hard-liner 200 with the shell 60. Hard-liners are generally known in the art and may include a male taper formed on the liner to engage a female taper in the shell, such as the C2a-Taper™ prosthesis system sold by Biomet, Inc. Accordingly, a description of the hard-liner and the shell taper configuration is not specifically included herein. Nevertheless, the multi-use tool 20 can be used to engage the hard-liner 200, as discussed further herein.

Initially, with continuing reference to Fig. 4, the multi-use tool 20 can be used to engage the hard-liner 200. As discussed above, the multi-use tool 20 can include at least the non-metallic buffer pads 90a-90c to contact an upper rim 210 of the hard-liner 200. The non-metallic buffer pads 90a-90c can be formed of a material that is softer than the hard-liner 200 and generally less able or likely to scratch or form debris when engaging the hard-liner 200, relative to the other portions of the multi-use tool 20. For example, various polymers can be used as a buffer or cushioning material for the buffers 90a-90c. The buffers 90a-90c can be positioned to engage the rim 210 of the hard-liner 200 generally internally relative to the ring 24 of the multi-use tool 20. As discussed in Figs. 1A-1B and further illustrated in Fig. 4, the side 50 of the multi-use tool 20 that includes the buffers 90a-90c is opposite of the side 40 that engages the shell 60. Accordingly, the multi-use tool can be used to engage both the hard-liner 200 and the shell 60 for positioning of the shell 60 within the anatomy and/or the hard-liner 200 within the shell 60.

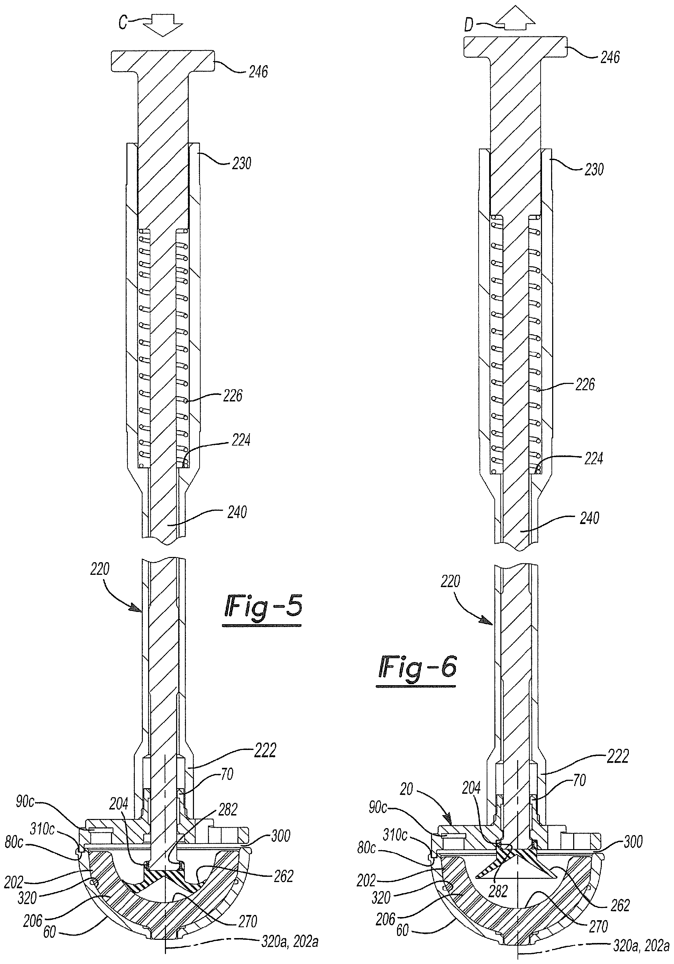

The multi-use tool 20, as discussed above, can include the threaded hub 70 that can engage a thread of a sleeve 220. The thread of the sleeve 220 can be an internal or an external thread to mate with an internal or external thread, respectively, of the hub 70. Accordingly, an external thread on the hub 70 and internal thread on the sleeve 220 is not required, but exemplarily illustrated herein. The sleeve 220 engages the multi-use tool 20 at a first end 222. The sleeve 220 may define an internal bore or cannula that has a shoulder 224, as illustrated in Fig. 5. The shoulder 224 may hold a spring 226 spaced a distance away from the first end 222 of the sleeve 220. It is understood, however, that the shoulder 224 can be positioned at any point along the sleeve 220. Having the shoulder 224 spaced a distance from the first end 222 can be selected for various purposes, such as manufacturing, positioning a second end 230 a selected distance from the first end 222 for use by a user, or other appropriate reasons.

The spring 226 can be pressed against the shoulder 224 by an internal rod 240. The internal rod 240 can include a second shoulder 242 that engages the spring 226 to compress it against the first shoulder 224. A user can press a plunger end 246 to compress the spring 226 against the first shoulder 224. By compressing the spring 226 against the first shoulder 224, the rod 240 may move generally in the direction of Arrow C to move a first rod end 250 relative to the first sleeve end 222. The spring 226 may bias the internal rod 240 generally in a direction of Arrow D, which can generally be opposite to Arrow C. In turn, the internal rod 240 is biased towards the second end 230 of the sleeve 220.

As discussed above the sleeve 220 can engage the boss 70 of the multi-use tool 20. The rod 240 can pass through the sleeve 220 and also through the central bore of the multi-use tool 20. The rod 240, therefore, can engage a suction cup or attachment member 260. The suction cup member 260 can include a suction cup portion 262 that is formed as a suction cup member. The suction cup member 260 may be formed by silicone or rubberized polymer material that may deflect and engage the hard-liner 200. As is generally known in the art, the hard-liner 200 can include an internal surface that can be an internal articulating surface 270 that is substantially smooth. The suction cup or attachment member 262 may engage the internal surface 270 of the hard-liner 200 to hold the hard-liner 200 relative to the multi-use tool 20, such as with engagement to the buffer members 90a-90c. The multi-use tool 20 after having engaged the hard-liner 200 with the suction cup member 260 is illustrated in Fig. 5. Illustrated in Fig. 6 is after the suction cup member 260 is disengaged from the hard-liner 200, which may be after the hard-liner 200 has been positioned in the shell 60.

The engaging member 260 can include a rod connection portion 280 which may include an internal thread 282 to engage an external thread 284 of the rod 240. It is understood that other connections between the rod 240 and the liner engaging member 260 may also be provided. For example, a snap fit, a friction/interference connection, or other connections can also be provided. Nevertheless, the assembly can be formed such that the sleeve 220 can engage the multi-use tool 20 with a first connection and the rod 240 can pass through the sleeve 220 and the multi-use tool 20. Once the rod 240 is placed through the sleeve 220 and tool 20 it may engage the liner engaging member 260. Moving the internal rod 240 relative to the sleeve 220 may allow the suction member 260 to press against and engage the liner 200. Once engaged with the engagement member 260, the rod 240 can be released and the spring may then bias the rod 240 and move the hard-liner 200 against the multi-use tool 20, as illustrated in Fig. 5. Once the liner 200 is engaged against the multi-use tool 20, the liner 200 can be positioned within the acetabular shell 60. It is understood that the acetabular shell 60 can be positioned within the acetabulum 110 of the pelvis prior to positioning the liner 200 within the shell 60.

As illustrated in Figs. 5 and 6, the multi-use tool 20 can engage the shell 60 such that there is a gap 300 between the ring 24 and the upper rim of the shell 60. The gap 300 is due to the length of the tabs 80a-80b that have engaged respective pockets 310 of the shell 60. It is understood that a respective number or complementary number of pockets are formed in the shell 60 based upon the number of tabs 80 of the multi-use tool 20, and only one is illustrated here for the purposes of the current disclosure. Nevertheless, any appropriate number such as 2, 3, 4 or more may be provided. Nevertheless, the tabs 80a-80c can engage their respective pockets 310a-310c to hold the ring 24 of the multi-use tool 20 a selected distance away from the shell 60. The tabs 80a-80c, however, may bottom out and engage the pockets 310a-310c of the shell 60 to substantially ensure that the ring 24 of the multi-use tool 20 is substantially square or aligned with the shell 60.

The squaring of the multi-use tool 20 with the shell 60 can include ensuring that the male taper 202 of the liner 200 is square or aligned with a female taper 320 of the shell 60. The female taper 320 can define a central or perpendicular axis 320a that may be aligned with a central or perpendicular axis 202a of the male taper 202 at insertion of the hard-liner 200 into the shell 60. By ensuring that the axes 320a and 202a are substantially aligned, the liner 200 may seat properly and fully within the shell 60. By ensuring a proper seat of the liner 200 within the shell 60, a substantially reduced possibility of fracture or breaking of the liner 200 can occur. Accordingly, the liner 200 can be engaged against the multi-use tool 20, via the buffer members 90a-90c, to ensure that the liner 200 is square with the multi-use tool 20. The multi-use tool 20 can then engage the shell 60 via the tabs 80a-80c and the pockets 310a-310c to ensure that the multi-use tool 20 is square and aligned with the shell 60. In this way, as the liner 200 is square and aligned with a multi-use tool 20, and the multi-use tool 20 is square and aligned with the shell 60, the liner 200 is squared and aligned with the shell 60.

As illustrated in Fig. 5, once the shell is aligned with the liner 200, the central rod 240 can be compressed, generally in the direction of Arrow C, to move the liner 200 into the shell 60. Once the liner 200 is engaged in the shell 60, the rod 240 can be released from the force that moves it in the direction of Arrow C. Removing the force will allow the spring 226 to bias the rod 240 generally in the direction of Arrow D, as illustrated in Fig. 6. As the liner 200 is seated within the shell 60, a force of the suction cup portion 262 of the engagement member 260 can be overcome by the biasing force of the spring 226 and the suction cup member 262 can disengage the liner 200, as illustrated in Fig. 6. The tabs 80a-80c will then be easily disengaged from the pockets 310a-310c of the shell 60. This can allow the liner 200 to be properly seated in a substantially squared and aligned manner and orientation with the shell 60 during positioning of the liner 200 within the shell 60.

In addition to insertion of the hard-liner 200, with the tabs 80a-80c, the multi-use tool 20 can assist in removal of the hard-liner 200 as well. Once the hard-liner 200 is seated and engaged in the shell 60, the taper of the hard-liner 200 is engaged with the taper of the shell 60. A selected vibration induced in the shell 60, however, can disengage the hard-liner 200 from the taper connection with the shell 60.

To remove the hard-liner, the multi-use tool 20 can engage the shell 60 via the tabs 80a-80c in the pockets 310a-310c similar or identical to the orientation illustrated in Fig. 5. A handle, such as the shell insertion handle 72, can engage the multi-use tool 20. The insertion handle 72 can then be impacted with the impaction instrument 120 to cause vibration within the shell 60. The shell 60, having been implanted in the patient, can have a vibration induced therein without affecting positioning of the shell 60 within the patient. The vibration induced in the shell 60, however, can affect the hard-liner 200 that has been positioned within the shell 60. Vibrations induced within the shell 60 can cause the taper connection or taper lock between the hard-liner 200 and the shell 60 to disengage.

Once the hard-liner 200 is disengaged from the taper connection, the hard-liner 200 can be removed from the shell 60. Removal of the hard-liner 200 from the shell 60 can be according to various mechanisms such as manual manipulation with a digit of a user or a grasping tool. Additionally, a suction cup member, similar to that used for insertion of the hard-liner 200, can be used to engage the hard-liner 200 to remove it from the shell 60. Accordingly, the multi-use tool 20 can be used for both insertion and removal of the hard-liner 200 from the shell 60.

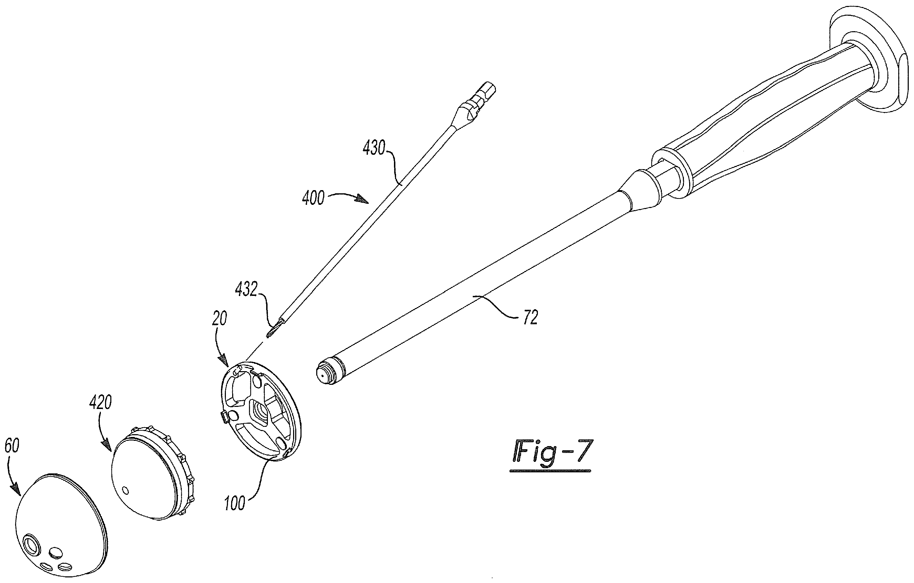

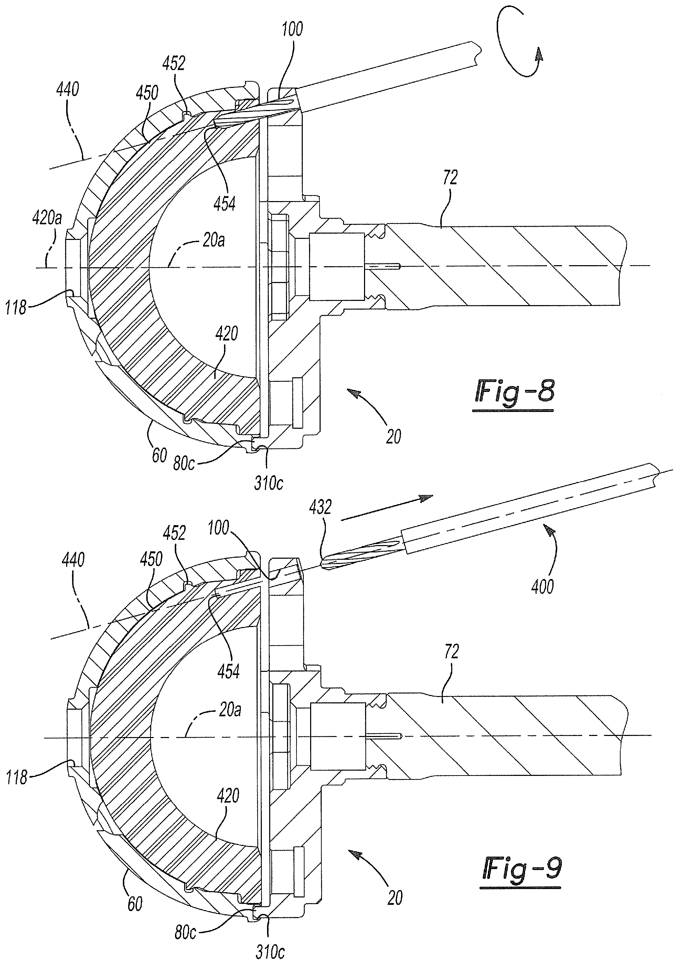

The multi-use tool 20, as illustrated in Fig. 7 can be used in conjunction with a pilot bore drill 400 to form a pilot hole in a soft-liner 420 that has been positioned in the shell 60. The soft liner 420 can be formed of a selected material, such as a polymer, including a high molecular weight polyethylene, or other selected material generally used for forming acetabular bearing liners of the shell 60 for an acetabular prosthesis. The soft-liner can be formed to have a central axis 420a. The pilot hole drill 400 can include a drill shaft 430 and a drill tip 432. The drill tip 432 can be of a selected length to pass through the guide-bore 100 of the multi-use tool 20 to engage the soft liner 420 to form a pilot bore 454 (illustrated in Figs. 8 and 9) therein. For example, the drill tip 432 can include a length to extend through the multi-use tool 20 and engage the soft liner 420 for a selected distance, such as about 3 mm to about 10 mm. In addition, the guide-bore 100 can include a longitudinal axis 440 that is formed at an angle to a central axis 20a of the multi-use-tool 20 and/or the soft-liner axis 420a. Therefore, when the multi-use tool 20 is positioned on the shell 60, as illustrated in Fig. 8, the axis 440 of the guide-bore 100 is angled relative to the central axis of the multi-use tool 20. Again, the multi-use tool 20 may engage the shell 60 via the tabs 80a-80c that engage the pockets 310a-310c of the shell 60 at the rim. The multi-use tool 20 can be positioned relative to the shell 60 with the insertion rod 72 or any other selected member that can engage the multi-use tool 20.

The guide-bore 100 can allow the drill bit tip 432 to engage the soft liner 420 along the axis 440 of the guide-bore 100 such that a straight line along the axis 440 engages the interior surface of the shell 60 at a point 450 closer to the apical hole 118 than a locking or engagement groove 452 within the shell 60. Accordingly, the axis 440 intersects the interior surface of the shell 60 in a position that does not interfere with any locking portions of the shell 60. Generally, a taper locking portion to engage a hard-liner is also positioned further toward the rim of the shell 60 than the apical hole 118. Thus, the pilot bore 454 can be formed in the soft liner 420 using the drill tip 432 through the guide-bore 100.

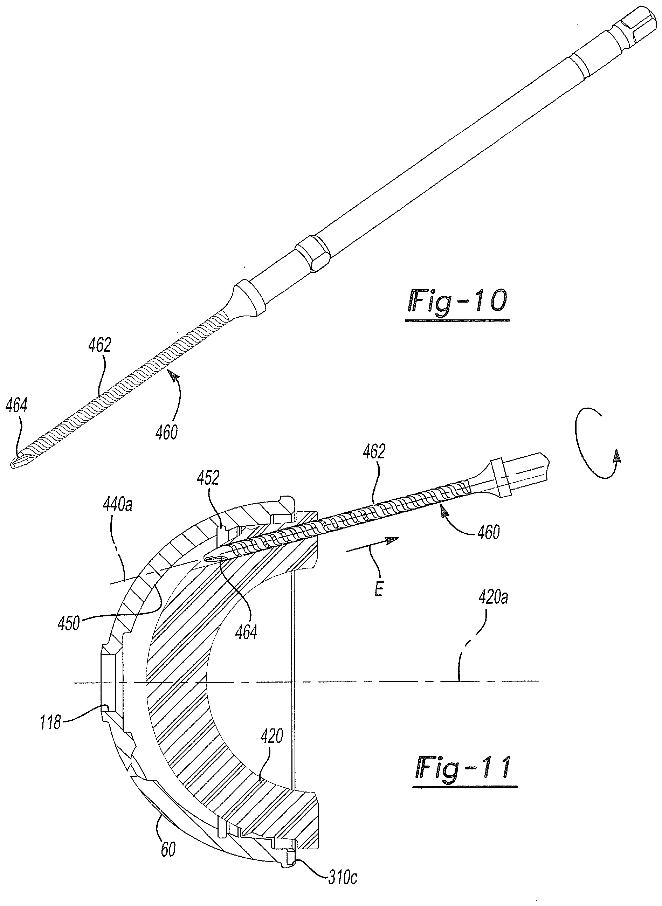

Once the pilot bore 454 is formed within the soft liner 420, the drill 400 can be removed, with reference to Fig. 9, and the multi-use tool 20 can also be removed from the shell 60. Turning reference to Figs. 10 and 11, the pilot bore 454 formed within the soft liner 420 can guide a soft liner removal tool 460 into the pilot bore 454 generally along the axis 440a now defined by the pilot bore 454. The axis 440a, being substantially identical to the axis 440 of the pilot bore 454, ensures that the liner removal tool 460 engages the shell 60 at the position 450 spaced away from the locking groove 452 of the shell 60.

The removal tool 60 can include a thread or spiral groove 462 that can engage the liner 420 during insertion of the liner removal tool 460. The removal tool 460 can also include a sharpened tip 464 that can further engage and pass through the soft liner 420. As the removal tool 460 moves along the axis 440a, it will engage the shell 60 at or near the point 450. Due to the threads 462 on the removal tool 460, the liner 420 will begin to move generally in the direction of Arrow E towards the rim and away from the apical hole 118 of the shell 60. This allows the liner 420 to be removed from the shell 60 due to movement along the removal tool 460. The movement of the liner 420 will disengage any liner engagement or locking mechanism with the shell 60 and the liner 420 can then be removed from the shell 60, as illustrated in Fig. 11.

The removal tool 460 engages the shell 60 away from regions where a liner would engage and be locked into the shell 60. Because the removal tool 460 engages the shell 60 away from locking portions for the liner 420 or the hard-liner 200, a new liner or revision liner can be positioned within the shell 60 without requiring replacement of the shell 60. The guide-bore 100 allows for the formation of the pilot hole 454 in a selected orientation relative to the shell 60 to ensure that the removal tool 460 does not engage the shell 60 at a position that would damage or affect locking portions of the liner and shell 60 engagement.

The foregoing description of the embodiments has been provided for purposes of illustration and description. It is not intended to be exhaustive or to limit the disclosure.

## Claims

1. An instrument assembly for an acetabular prosthesis system, comprising:
a member (20) having a first side (40) having a first engaging projection (56a-56c) configured to engage a complementary recess (58) of an acetabular shell (60) to rotationally hold the acetabular shell relative to the member;
the member defining a guide-bore (100) through the member, wherein the guide-bore is configured to guide a bit (432) through the guide-bore and into a soft bearing liner placed within the acetabular shell;
wherein the guide-bore (100) is formed at a selected angle through the member along a guide-bore axis (440), wherein the guide-bore axis intersects an internal surface of the acetabular shell at a distance from a bearing liner connection section, **characterized in that**
the member (20) is configured to be placed in a first orientation relative to the acetabular shell to implant the acetabular shell within a subject and then placed in a second orientation to position the guide-bore relative to the soft bearing liner placed within the acetabular shell to form a pilot bore (454) within the soft bearing liner and
the member further includes tabs (80a-80c) extending from a second side (50) to engage the acetabular shell to selectively fix the member relative to the acetabular shell during formation of the pilot bore.

2. The instrument assembly of Claim 1, further comprising:
a handle (72);
wherein the handle is configured to engage the member (20) on the second side (50) while the member engages the acetabular shell (60) with the first side (40);
wherein the handle (72) is configured to disengage from the second side (50) and engage the first side (40) when forming the pilot bore into the soft bearing liner.

3. The instrument assembly of Claim 1, further comprising:
a bearing liner removal bit (460) having a spiral thread formed along a length of the bearing liner removal bit;
wherein the bearing liner removal bit is configured to be driven into the pilot hole (454) and contact the internal surface of the acetabular shell (60) at the distance from the bearing liner connection section;
wherein rotation of the bearing liner removal bit while engaging the soft bearing liner and contacting the internal surface of the acetabular shell urges the soft bearing liner to move along the bearing liner removal bit.

4. The instrument assembly of Claim 3, further comprising:
the acetabular shell (60) configured to be implanted with the member;
a soft bearing liner (420) configured to be removed from the acetabular shell (60) with use of the guide-bore.

5. The instrument assembly of Claim 3, further comprising:
a hard bearing liner (200), wherein the hard bearing liner is formed of a material harder than the soft bearing liner (420);
a handle (72);
an outer sleeve (220),
a spring (226) within the outer sleeve,
a rod (240) moveable within the outer sleeve and biased in a first direction relative to the outer sleeve with the spring, and
a hard bearing liner engagement member (90a-90c) configured to selectively hold the hard bearing liner relative to the rod;
wherein the handle is configured to engage the member (20) on the second side (50) while the member engages the acetabular shell (60) with the first side (40);
wherein the handle is configured to disengage from the second side (50) and engage the first side (40) to insert the hard bearing liner with the member;
wherein the outer sleeve is configured to engage the member and the rod is configured to further move through the member to engage and disengage the hard bearing liner engagement member from the hard bearing liner;
wherein the member further includes tabs (80a-80c) extending from the second side to engage the acetabular shell to align the member with the acetabular shell.

6. The instrument assembly of Claim 1, further comprising:
a handle (72) configured to engage the member (20) on the second side (50) while the member engages the acetabular shell (60) with the first side (40);
a connection member (260) extending from the member to engage the acetabular shell with a snap fit.

## Patentansprüche

1. Instrumentenanordnung für ein Hüftgelenkprothesensystem, umfassend:
ein Element (20), das eine erste Seite (40) aufweist, die einen ersten Eingriffsvorsprung (56a - 56c) aufweist, der dazu ausgestaltet ist, mit einer komplementären Aussparung (58) einer Hüftgelenkpfanne (60) in Eingriff zu kommen, um die Hüftgelenkpfanne relativ zum Element drehbar zu halten;
wobei das Element eine Führungsbohrung (100) durch das Element definiert, wobei die Führungsbohrung dazu ausgestaltet ist, einen Einsatz (432) durch die Führungsbohrung und in eine weiche Lagereinlage zu führen, die im Inneren der Hüftgelenkpfanne angeordnet ist;
wobei die Führungsbohrung (100) in einem ausgewählten Winkel durch das Element entlang einer Führungsbohrungsachse (440) ausgebildet ist, wobei die Führungsbohrungsachse eine Innenfläche der Hüftgelenkpfanne in einem Abstand von einem Lagereinlageverbindungsabschnitt schneidet, **dadurch gekennzeichnet, dass** das Element (20) dazu ausgestaltet ist, in einer ersten Ausrichtung relativ zur Hüftgelenkpfanne angeordnet zu werden, um die Hüftgelenkpfanne im Inneren einer Person zu implantieren, und dann in einer zweiten Ausrichtung angeordnet zu werden, um die Führungsbohrung relativ zur weichen Lagereinlage zu positionieren, die im Inneren der Hüftgelenkpfanne angeordnet ist, um eine Pilotbohrung (454) im Inneren der weichen Lagereinlage zu bilden, und das Element ferner Laschen (80a - 8oc) umfasst, die sich von einer zweiten Seite (50) aus erstrecken, um mit der Hüftgelenkpfanne in Eingriff zu kommen, um das Element selektiv relativ zu der Hüftgelenkpfanne während der Bildung der Pilotbohrung zu fixieren.

2. Instrumentenanordnung nach Anspruch 1, ferner umfassend:
einen Griff (72);
wobei der Griff dazu ausgestaltet ist, mit dem Element (20) auf der zweiten Seite (50) in Eingriff zu kommen, während das Element mit der ersten Seite (40) in Eingriff mit der Hüftgelenkpfanne (60) steht;
wobei der Griff (72) dazu ausgestaltet ist, sich von der zweiten Seite (50) zu lösen und mit der ersten Seite (40) in Eingriff zu kommen, wenn die Pilotbohrung in die weiche Lagereinlage geformt wird.

3. Instrumentenanordnung nach Anspruch 1, ferner umfassend:
einen Lagereinlageentfernungseinsatz (460), der ein spiralförmiges Gewinde aufweist, das entlang einer Länge des Lagereinlageentfernungseinsatzes ausgebildet ist;
wobei der Lagereinlageentfernungseinsatz dazu ausgestaltet ist, in die Pilotbohrung (454) eingetrieben zu werden und die Innenfläche der Hüftgelenkpfanne (60) im Abstand vom Lagereinlageverbindungsabschnitt zu berühren;
wobei die Rotation des Lagereinlageentfernungseinsatzes, während er mit der weiche Lagereinlage in Eingriff steht und die Innenfläche der Hüftgelenkpfanne berührt, die weiche Lagereinlage dazu drängt, sich entlang des Lagereinlageentfernungseinsatzes zu bewegen.

4. Instrumentenanordnung nach Anspruch 3, ferner umfassend:
die Hüftgelenkpfanne (60), die dazu ausgestaltet ist, mit dem Element implantiert zu werden; eine weiche Lagereinlage (420), die dazu ausgestaltet ist, unter Verwendung der Führungsbohrung von der Hüftgelenkpfanne (60) entfernt zu werden.

5. Instrumentenanordnung nach Anspruch 3, ferner umfassend:
eine harte Lagereinlage (200), wobei die harte Lagereinlage aus einem Material gebildet ist, das härter ist als die weiche Lagereinlage (420);
einen Griff (72); eine Außenhülse (220), eine Feder (226) im Innern der Außenhülse, eine Stange (240), die im Inneren der Außenhülse beweglich und in einer ersten Richtung relativ zur Außenhülse mit der Feder vorgespannt ist, und ein Eingriffselement (90a-90c) der harten Lagereinlage, das dazu konfiguriert ist, selektiv die harte Lagereinlage zu der Stange zu halten;
wobei der Griff dazu ausgestaltet ist, mit dem Element (20) auf der zweiten Seite (50) in Eingriff zu kommen, während das Element mit der ersten Seite (40) in Eingriff mit der Hüftgelenkpfanne (60) steht;
wobei der Griff dazu ausgestaltet ist, sich von der zweiten Seite (50) zu lösen und mit der ersten Seite (40) in Eingriff zu kommen, um die harte Lagereinlage mit dem Element einzusetzen;
wobei die Außenhülse dazu ausgestaltet ist, mit dem Element in Eingriff zu kommen, und die Stange dazu ausgestaltet ist, sich weiter durch das Element zu bewegen, um das Eingriffselement der harten Lagereinlage mit der harten Lagereinlage in Eingriff zu bringen und davon zu lösen;
wobei das Element ferner Laschen (8oa-8oc) umfasst, die sich von der zweiten Seite aus erstrecken, um mit der Hüftgelenkpfanne in Eingriff zu kommen, um das Element an der Hüftgelenkpfanne auszurichten.

6. Instrumentenanordnung nach Anspruch 1, ferner umfassend:
einen Griff (72), der dazu ausgestaltet ist, mit dem Element (20) auf der zweiten Seite (50) in Eingriff zu kommen, während das Element mit der ersten Seite (40) in Eingriff mit der Hüftgelenkpfanne (60) steht;
ein Verbindungselement (260), das sich von dem Element aus erstreckt, um die Hüftgelenkpfanne mit einer Schnappverbindung in Eingriff zu bringen.

## Revendications

1. Ensemble d'instrument pour un système de prothèse acétabulaire, comprenant :
un élément (20) ayant un premier côté (40) ayant une première saillie d'engagement (56a-56c) configurée pour s'engager dans un creux complémentaire (58) d'une coquille acétabulaire (60) pour maintenir la coquille acétabulaire en rotation par rapport à l'élément ;
l'élément définissant un alésage-guide (100) à travers l'élément, l'alésage-guide étant configuré pour guider une mèche (432) à travers l'alésage-guide et dans une doublure porteuse tendre placée à l'intérieur de la coquille acétabulaire ;
dans lequel l'alésage-guide (100) est formé suivant un angle sélectionné à travers l'élément le long d'un axe d'alésage-guide (440), dans lequel l'axe de l'alésage-guide coupe une surface interne de la coquille acétabulaire à une distance de la section de raccordement de doublure porteuse, **caractérisé en ce que**
l'élément (20) est configuré pour être placé dans une première orientation relative à la coquille acétabulaire pour implanter la coquille acétabulaire dans un sujet, puis placé dans une deuxième orientation pour positionner l'alésage-guide par rapport à la doublure porteuse tendre dans la coquille acétabulaire pour former un alésage pilote (454) dans la doublure porteuse tendre et l'élément comporte en outre des languettes (80a-80c) s'étendant d'un deuxième côté (50) pour s'engager avec la coquille acétabulaire afin de fixer sélectivement l'élément par rapport à la coquille acétabulaire pendant la formation de l'alésage pilote.

2. Ensemble d'instrument selon la revendication 1, comprenant en outre :
une poignée (72) ;
dans lequel la poignée est configurée pour s'engager avec l'élément (20) sur le deuxième côté (50) tandis que l'élément s'engage avec la coquille acétabulaire (60) du premier côté (40) ;
dans lequel la poignée (72) est configurée pour se désengager du deuxième côté (50) et s'engager avec le premier côté (40) lors de la formation de l'alésage pilote dans la doublure porteuse tendre.

3. Ensemble d'instrument selon la revendication 1, comprenant en outre :
une mèche d'enlèvement de doublure porteuse (460) ayant un filetage en spirale formé sur une longueur de la mèche d'enlèvement de doublure porteuse ;
dans lequel la mèche d'enlèvement de doublure porteuse est configurée pour être enfoncée dans le trou pilote (454) et entrer en contact avec la surface interne de la coquille acétabulaire (60) à la distance de la section de raccordement de la doublure porteuse ;
dans lequel la rotation de la mèche d'enlèvement de la doublure porteuse pendant l'engagement de la doublure porteuse tendre et l'entrée en contact de la surface interne de la coquille acétabulaire force la doublure porteuse tendre à se déplacer le long de la mèche d'enlèvement de la doublure porteuse.

4. Ensemble d'instrument selon la revendication 3, comprenant en outre :
la coquille acétabulaire (60) configurée pour être implantée avec l'élément ;
une doublure porteuse tendre (420) configurée pour être enlevée de la coquille acétabulaire (60) au moyen de l'alésage-guide.

5. Ensemble d'instrument selon la revendication 3, comprenant en outre :
une doublure porteuse dure (200), la doublure porteuse dure étant constituée d'un matériau plus dur que la doublure porteuse tendre (420) ;
une poignée (72) ;
un manchon extérieur (220),
un ressort (226) à l'intérieur du manchon extérieur,
une tige (240) mobile à l'intérieur du manchon extérieur et rappelée dans une première direction par rapport au manchon extérieur avec le ressort, et
un élément d'engagement de doublure porteuse dure (90a-90c) configuré pour retenir sélectivement la doublure porteuse dure par rapport à la tige ;
dans lequel la poignée est configurée pour s'engager avec l'élément (20) du deuxième côté (50) tandis que l'élément engage la coquille acétabulaire (60) avec le premier côté (40) ;
dans lequel la poignée est configurée pour se désengager du deuxième côté (50) et s'engager avec le premier côté (40) pour insérer la doublure porteuse dure avec l'élément ;
dans lequel le manchon extérieur est configuré pour s'engager avec l'élément et la tige est configurée pour se déplacer davantage à travers l'élément pour engager et désengager l'élément d'engagement de doublure porteuse dure de la doublure porteuse dure ;
dans lequel l'élément comporte en outre des languettes (80a-80c) s'étendant du deuxième côté pour s'engager avec la coquille acétabulaire afin d'aligner l'élément avec la coquille acétabulaire.

6. Ensemble d'instrument selon la revendication 1, comprenant en outre :
une poignée (72) configurée pour s'engager avec l'élément (20) du deuxième côté (50) tandis que l'élément engage la coquille acétabulaire (60) avec le premier côté (40) ;
un élément de raccordement (260) s'étendant de l'élément pour s'engager avec la coquille acétabulaire par enclenchement.
